# EUROPEAN PATENT SPECIFICATION

(11) **EP 0 765 276 B1**
(45) Date of publication and mention of the grant of the patent: **24.10.2001**
(21) Application number: 95924607.5
(22) Date of filing: 22.06.1995
(51) Int. Cl.: B65D 47/18, B65D 51/22

(54) **NON-STREAMING OPHTHALMIC TIP AND DELIVERY DEVICE**
OPHTHALMOLOGISCHE AUSGABESPITZE UND AUSGABEVORRICHTUNG ZUR TROPFENWEISEN ABGABE
EMBOUT DE DISTRIBUTION POUR PREPARATION OPHTALMIQUE, NE CREANT PAS D'ECOULEMENT CONTINU ET DISPOSITIF DISTRIBUTEUR ASSOCIE

(30) Priority: 24.06.1994 US 265351
(43) Date of publication of application: 02.04.1997
(73) Proprietor: Merck & Co., Inc., Rahway New Jersey 07065-0900 (US)
(72) Inventor: LIFSHEY, Arthur, L., Rahway, NJ 07065 (US)
(74) Representative: Thompson, John Dr.
(86) International application number: PCT/US95/07708
(87) International publication number: WO 96/00173

(56) References cited:
- EP-A- 0 228 751
- FR-A- 2 540 071
- IT-A- 372 497
- US-A- 2 411 435
- US-A- 2 671 577
- US-A- 5 052 589

## Description

### TECHNICAL FIELD

This invention relates to a liquid dispensing tip and cap especially useful in the dispensing of ophthalmic drugs which typically need to be dispensed in the form of a drop, and a novel method of manufacturing the device.

### BACKGROUND OF THE INVENTION

The present invention pertains to the art of liquid dispensers, and, more particularly, to a dispensing tip for accurately dispensing small droplets of liquid. The invention is particularly applicable for use as an eyedropper to dispense ophthalmic drugs and will be described with particular reference thereto although it will be appreciated that the invention has other and broader applications.

Medicant drop dispensers of the type to which the present invention pertains are available in various sizes and shapes for the numerous medicines and solutions which are available for the care and comfort of the human eye. Such dispensers are basically comprised of a relatively small compressible plastic container or vial provided with a dispensing tip and cap.

One problem associated with conventional eyedroppers is the difficulty in accurately controlling the amount of medicine dispensed, i.e., the number of drops dispensed. Many conventional eyedroppers utilize one or a combination of methods to achieve single drop control.

One method uses a highly compliant plastic bottle which the user squeezes to dispense a drop. The extensive deflection of the bottle creates an significant internal air pressure within the vial which expels the liquid through the tip. In order to prevent a continuous stream of liquid medicant from being expelled, and to create single drops, these bottles sometimes incorporate a flow restriction at the inlet of the tip or nozzle. This flow restriction tends to limit the number of drops expelled during a single squeeze. It limits the liquid medicant flow rate favoring the formation of individual drops releasing from the dropper tip rather than a continuous stream. Unfortunately, the creation of a very small molded orifice, frequently as small as .005" (.13 mm) in diameter creates manufacturing difficulties, since the plastic injection molds must have corresponding small fragile (.005" dia./.13 mm dia.) core pins.

### SUMMARY OF THE INVENTION

The present invention discloses an ophthalmic tip and cap system providing nonstreaming drop control, the tip having an internally molded dislodgeable barrier which may be hinged on one side, the cap having a stud shaped to fit inside the nozzle tip, so that the downward axial movement of the cap either partially or completely displaces the barrier, wherein nonstreaming drop control results when the barrier is captivated in its new open position allowing the barrier to function as a flow restrictor able to resist the internal pressure generated in the vial during dispensing. The tip may have molded flow restriction groove(s) or slot(s) on its internal flow channel. The cap of the device is designed such that upon downward motion of the cap, the tip barrier is partially displaced resulting in subsequent mechanical captivation of the barrier in its open position to create a flow restriction. The cap may also be designed so that the tip barrier is completely displaced by the downward movement of the cap and subsequent mechanical captivation of the barrier in its open position to create a flow restriction.

### BRIEF DESCRIPTION OF THE DRAWINGS

Fig 1 a cross-sectional view of the tip affixed to a bottle and the mating cap in the unactivated position where the barrier membrane 5 is angled relative to the activating stud (6).
Fig 1(a) is a cross-sectional view of the tip affixed to a bottle and the mating cap in the unactivated position where the barrier membrane 50 is circular in shape and lies parallel to the face of the activating stud (6) and perpendicular to the tip (1).
Fig 1(b) is a cross-sectional view of the tip affixed to a bottle and the mating cap in the unactivated position where the barrier membrane 51 is spherical in shape and is removably attached to the tip (1).
Fig 2 is a cross-sectional view through section 2-2 of Fig. 1.
Fig 2(a) is a cross-sectional view through section 2-2 showing four slots(8) rather than a single slot (8).
Fig 2(b) is a cross-sectional view through section 2-2 showing a plurality of slots (8).
Fig. 3 is a cross sectional of view through the tip and a portion of the cap which shows the barrier after being activated.
Fig. 3(a) and Fig 3(b) show the device of Fig 1(a) and Fig 1(b) in the activated position where the barrier 50 and 51 are totally dislodged from their original position.
Fig. 4 is a cross sectional view of the tip in the inverted position with the flow path (10) indicated.
Fig 4(a) and Fig (4(b) are cross sectional views of the tip in the inverted position with the flow path (10) indicated for the device as shown in Fig 1(a) and Fig 1(b) respectively.
Fig. 5 is a side view and partial cross sectional view of the tip when integrally molded as part of an injection molded vial for the delivery of medicament.

### DETAILED DESCRIPTION OF THE INVENTION

The present invention contemplates a new and improved tip and cap for ophthalmic use, which overcomes the above-referred to manufacturing difficulties and which accurately dispenses droplets of liquid medicant at desired locations as discrete drops. Single drops having a volume of approximately 30-50 microliters are typical in the art, but larger or smaller drops, can be provided by adjusting size and geometry of the tip.

In accordance with the present invention there is provided an improved tip (1), or drop dispenser for use with a closed compressible container. The preferred tip (1) is integrated into a simple one piece molded device, together with a mated cap (2) which is best understood by reference to the accompanying drawings. The tip/cap combination also serves as a means for providing an hermetically sealed tip which can be activated (opened) only when ready for use. This can be accomplished by only partially assembling the cap (2) onto the tip (1), so that the cap (2) does not displace the membrane (5) or barrier (5), (50) or (51) in the tip (1). The tip thus remains hermetically sealed, eliminating the possibility of minor leaks of liquid or vapor during long storage periods.

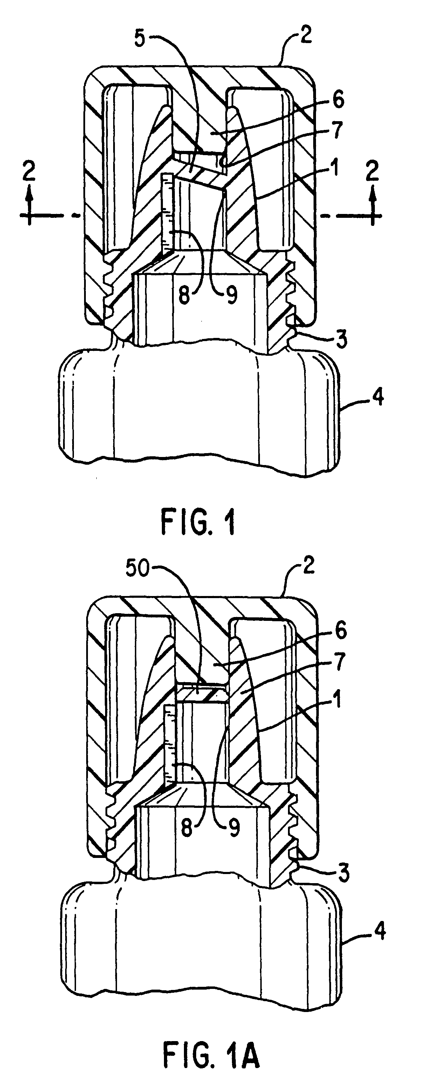

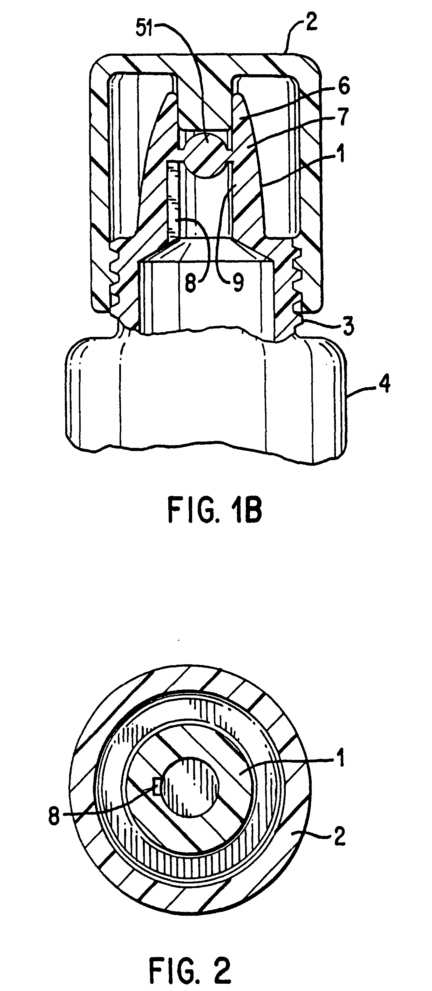

Figure 1 is a vertical sectional view of the one-piece molded tip having a cap with screw threads engaged around the tip.

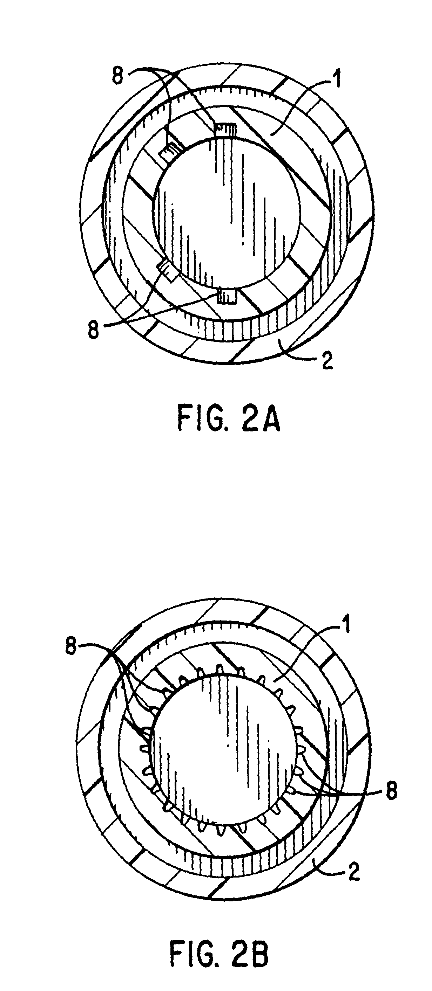

Figure 2 is a cross-section of Figure 1 taken along line 2-2, showing a restricted flow channel side slot. Figures 2a and 2b are alternate embodiments showing other restricted slot(s) or groove(s).

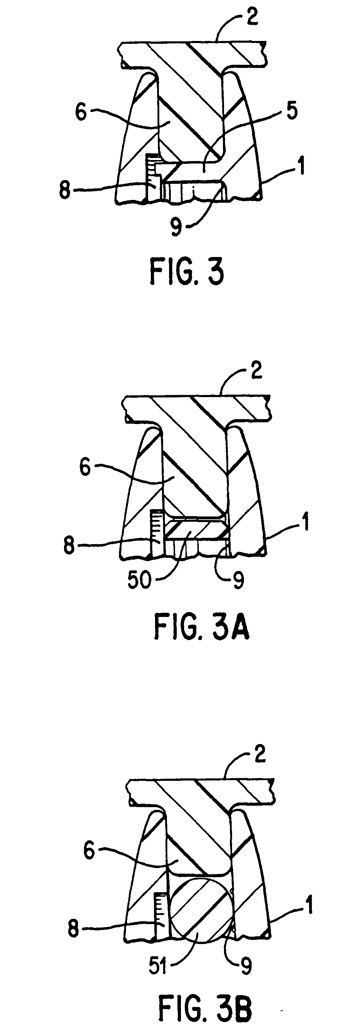

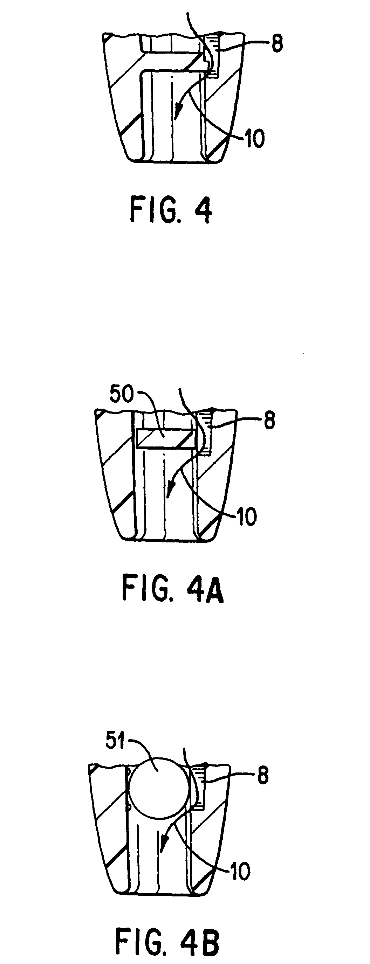

Figures 3 and 4 are vertical sectional views of the tip showing the action in opening the cap and the restricted flow path, which prevents streaming.

Figures 3A, 4A and 3B, 4B each show additional embodiments of this device.

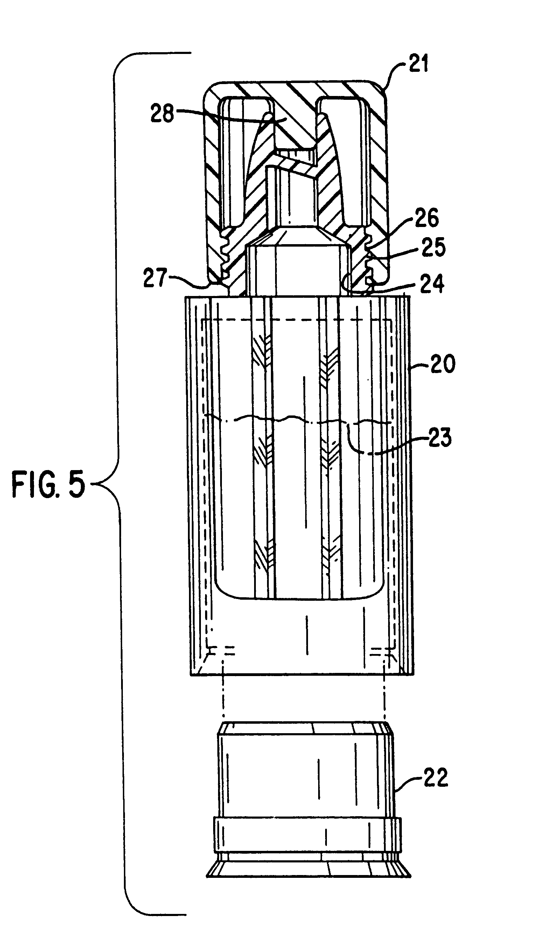

Figure 5 is a diagrammatic view of an example of an ophthalmic container having the tip with cap of this invention in place.

The actual bottle design is not a critical part of this invention. Any of the ophthalmic containers presently available can be used with the tip of this invention. The bottle design illustrated, which is especially preferred, is one having a limited displacement area, such as described in the invention claimed in U.S.S.N. 08/200,676, filed Dec. 22, 1993, attorney Docket No. 19140.

Referring to the drawings, the key aspects of this invention are described as follows.

In Figure 1, the ophthalmic tip, shown generally at (1), is protected by a cap (2), attached through screw threads (3). The bottle, which can be any type bottle useful for dispensing ophthalmic medicaments, is indicated generally at (4).

The cap (2) serves as a "seal-break". The tip is formed with a dislodgeable membrane or barrier (5) across the opening. The embodiment shown is eliptically shaped, and slanted so that it is attached higher inside the tip, at a point above a side slot (8). Other embodiments shown in Figure 1A and 1B can include a circular barrier (50) or spherical barrier (51) attached circumferentially to the inside wall of the tip (1). The inside of the cap (2) fitting over the nozzle opening is fitted with a suitably sized and shaped integrally molded stud (6). The cap (2) can be designed so that a final clockwise half-turn of the cap pushes the stud (6) against the barrier or membrane (5), (50) or (51) and downwardly displaces it while it remains attached at hinge point (9) to inside wall (7). Alternatively, the cap (2) and bottle (4) may be so designed so that the stud (6) is moved downward against barrier (5) by axial force applied to the cap. Other embodiment of this invention would have stud (6) completely dislodge barrier (50, 51) to a new position, which would break the hermetic seal and create a suitable flow restriction. In all cases, the barrier would be captivated in its new open position by friction fit, undercuts, or other mechanical means, allowing it to function as a flow restrictor able to resist the internal pressure generated in the vial during dispensing. Alternatively, the slot (8) in Fig. 2 may be of various sizes to allow for flow characteristics and viscosity of diverse fluids. Slot (8) may also consist of any number of shallow slots, as dictated by the flow properties.

The restricted flow channel side slot (8) is an important part of this invention, as it serves to aid in the prevention of a stream of medicament. Its shape in cross section is shown in Figures 2, and alternative embodiments shown in Fig. 2A and Fig. 2B wherein it can clearly be seen as molded slot(s) (8) or groove(s) in the nozzle channel.

Once the cap (2) is pushed or screwed down onto the tip (1), and the barrier (5) is displaced downwardly, the liquid contents can exit through the side slot (8) by flowing around the barrier (5), as shown in Figure 4, which is an upside-down vertical section. The arrow (10) in Figure 4 demonstrates the fluid path flow. The stud (6) of cap (2) is designed so that the stud (6) serves as a leak-proof closure for the bottle upon recapping after activation. Other embodiments shown in Figure 4A and 4B as previously discussed.

Another aspect of this invention which represents a significant improvement in the manufacturing process deals with the method of creating the flow restriction. The flow restriction is created when the liquid medicament must pass through an orifice with a very small area. For injection molding this usually means an opening created by a metal core pin with small diameter frequently as small as 0.13 mm and a corresponding length of 1 mm. Steel core pins of this size and slenderness are usually considered to be fragile cores, needing frequent maintenance and replacement. By contrast, this invention uses an open slot and a dislodgeable plastic member to create the flow restriction. Molding of a small slot has considerable advantage over molding a totally enclosed orifice, in that the very small portion of the core pin which forms the slot is fully attached to a larger core pin over its entire length and is no longer fragile.

The ophthalmic dispensing tip and cap of this invention can be injection molded of a suitable plastic, such as polyethylene or polypropylene.

In one preferred embodiment, the tip of this invention is molded as part of a bottle having a limited displacement side member as illustrated in Figure 5. The combination of a limited displacement bottle and the flow restriction in the instant dispensing tip are a way to prevent streaming. Figure 5 is a diagrammatic view of this embodiment of the ophthalmic container including the tip and cap of the instant invention. As noted, the invention of the bottle of Figure 5 is an independent invention, previously filed on Dec. 22, 1993, U.S.S.N. 08/200,676.

Figure 5 shows the tip and cap of the present invention in place on the bottle of the independent invention, comprised of a compressible plastic container or vial (20), a cap member (21), and a bottom closure (22). Container or vial (20) contains a supply of liquid (23), medicament for instance, to be dispensed in droplet form. The container (20) has an integral reduced diameter open neck portion (24) provided with external helical screw threads (25) over the uppermost part of the neck end portion as shown in Figure 5. The screw threads (25) are adapted to matingly engage internal threads (26) on cap member (21) to thereby attach the latter in place on container (20) in liquid-tight relation thereto.

Cap member (21) comprises a generally cylindrical mounting or base portion (27) and a inside nozzle end portion stud (28) projecting endwise therefrom.

## Claims

1. An ophthalmic tip and cap system providing nonstreaming drop control, the tip having an internally molded dislodgeable barrier which may be hinged on one side, the cap having a stud shaped to fit inside the nozzle tip, so that the downward axial movement of the cap either partially or completely displaces the barrier.

2. The tip of Claim 1, in which the tip has molded flow restriction groove(s) or slot(s) on the internal flow channel of the tip.

3. The cap of Claim 1 in which the tip barrier is partially displaced by the downward movement of the cap and subsequent mechanical captivation of the barrier in its open position to create a flow restriction.

4. The cap of Claim 1 in which the tip barrier is completely displaced by the downward movement of the cap and subsequent mechanical captivation of the barrier in its open position to create a flow restriction.

## Patentansprüche

1. Ophthalmo-Ausgabespitze- und Verschlusskappensystem, das für eine nicht fließende Tropfensteuerung sorgt, wobei die Ausgabespitze eine im Innern angeformte entfernbare Sperre aufweist, die auf einer Seite angelenkt sein kann, wobei die Verschlusskappe eine Nase aufweist, die so geformt ist, dass sie ins Innere der Düsenausgabespitze passt, so dass die axiale Abwärtsbewegung der Verschlusskappe die Sperre entweder teilweise oder ganz verlagert, wobei sich eine nicht fließende Tropfensteuerung ergibt, wenn die Sperre in ihrer neuen offenen Stellung festgehalten wird, was es ermöglicht, dass die Sperre als Durchflussbegrenzer wirkt, der dem während der Abgabe im Fläschchen erzeugten Innendruck widerstehen kann.

2. Ausgabespitze nach Anspruch 1, bei der die Ausgabespitze angeformte Durchflussbegrenzungsnut(en) oder -schlitz(e) auf dem inneren Strömungskanal der Ausgabespitze aufweist.

3. Verschlusskappe nach Anspruch 1, bei der die Ausgabespitzensperre durch die Abwärtsbewegung der Verschlusskappe und das anschließende mechanische Festhalten der Sperre teilweise in ihre offene Stellung verlagert wird, um eine Durchflussbegrenzung zu erzeugen.

4. Verschlusskappe nach Anspruch 1, bei der die Ausgabespitzensperre durch die Abwärtsbewegung der Verschlusskappe und das anschließende mechanische Festhalten der Sperre ganz in ihre offene Stellung verlagert wird, um eine Durchflussbegrenzung zu erzeugen.

## Revendications

1. Système d'embout et de bouchon ophtalmique fournissant une commande de goutte sans écoulement, l'embout ayant une barrière amovible moulée à l'intérieur qui peut être articulée sur un côté, le bouchon ayant un goujon mis en forme pour s'agencer à l'intérieur de l'embout de buse, de sorte que le déplacement axial vers le bas du bouchon déplace partiellement ou complètement la barrière, dans lequel la commande de goutte sans écoulement survient lorsque la barrière est fixée dans sa nouvelle position ouverte, en permettant à la barrière de fonctionner sous la forme d'un dispositif d'étranglement d'écoulement pouvant résister à la pression interne créée dans le flacon pendant une distribution.

2. Embout selon la revendication 1, dans lequel l'embout a une ou plusieurs gorges ou fentes d'étranglement d'écoulement moulées sur le canal d'écoulement intérieur de l'embout.

3. Bouchon selon la revendication 1, dans lequel la barrière d'embout est partiellement déplacée par le déplacement vers le bas du bouchon et une fixation mécanique consécutive de la barrière dans sa position ouverte crée un étranglement d'écoulement.

4. Bouchon selon la revendication 1, dans lequel la barrière d'embout est complètement déplacée par le déplacement vers le bas du bouchon et une fixation mécanique consécutive de la barrière dans sa position ouverte crée un étranglement d'écoulement.
